# FASCICULE DE BREVET EUROPEEN

(11) **EP 1 994 167 B1**
(45) Date de publication et mention de la délivrance du brevet: **31.08.2011**
(21) Numéro de dépôt: 07731707.1
(22) Date de dépôt: 08.03.2007
(51) Int. Cl.: C12Q 1/04, B01L 3/00

(54) **PROCEDE DE RELEVES D'IMAGES, NOTAMMENT POUR L'ETUDE DU DEVELOPPEMENT D'UN BIOFILM DANS UN MILIEU DE CULTURE**
VERFAHREN ZUM LESEN VON BILDERN, IM BESONDEREN ZUR UNTERSUCHUNG DER ENTWICKLUNG EINES BIOFILMS IN EINEM KULTURMEDIUM
METHOD FOR READING IMAGES, IN PARTICULAR FOR STUDYING THE DEVELOPMENT OF BIOFILM IN A CULTURE MEDIUM

(30) Priorité: 10.03.2006 FR 0602145
(43) Date de publication de la demande: 26.11.2008
(73) Titulaire: Biofilm Control, 63360 Saint Beauzire (FR)
(72) Inventeur: BERNARDI, Thierry, 63170 Perignat-les-sarliève (FR); BARA, Nicolas, 75002 Paris (FR)
(74) Mandataire: Novagraaf Technologies
(86) Numéro de dépôt international: PCT/FR2007/050891
(87) Numéro de publication internationale: WO 2007/104881

(56) Documents cités:
- EP-A1- 0 323 322
- WO-A-01/86255
- WO-A-97/12678
- WO-A-03/095995
- WO-A-2005/090944
- US-A- 3 635 678
- US-A1- 2001 033 414
- US-B1- 6 441 894

## Description

L'invention concerne le domaine de l'étude du comportement de cultures de microorganismes.

La présente invention se rapporte plus particulièrement, mais non exclusivement, au domaine de l'étude du développement d'un biofilm dans un milieu de culture homogène ou non homogène.

Par l'expression "milieu de culture", doit s'entendre tout milieu dans lequel au moins un microorganisme est susceptible d'être présent et de se développer. Il s'agit donc d'un milieu qui peut être naturel ou synthétique. Ainsi par exemple, l'eau entre dans cette définition. Ainsi, par "milieu de culture", on entend, selon l'invention, le microorganisme et le milieu dans lequel il se trouve, ou éventuellement le milieu seul.

Les biofilms sont formés de différentes couches de bactéries ou de microorganismes, contenus dans une matrice solide. Ils se développent pour former des communautés microbiennes, dont l'une des propriétés est d'adhérer aux surfaces submergées. Cette adhésion est soit non spécifique (adhérence), soit spécifique (adhésion proprement dite) (Costerton et al. Bacterial Biofilms. Sciences 1999 ; 284-6) :
- L'adhérence ou adhésion réversible : Les microorganismes présents se rapprochent des surfaces par gravimétrie, mouvements browniens ou par chimiotactisme s'ils possèdent des flagelles. Au cours de cette première étape de fixation, faisant intervenir uniquement des phénomènes purement physiques et des interactions physico-chimiques faibles, les microorganismes peuvent encore être facilement décrochés.
- L'adhésion : Cette étape plus lente fait intervenir des interactions de plus forte énergie ainsi que le métabolisme microbien et les appendices cellulaires du microorganisme (flagelles, pilis,....). L'adhésion est un phénomène actif et spécifique. Certains premiers colonisateurs vont s'attacher de manière irréversible à la surface grâce notamment à la synthèse d'exopolysaccharides (EPS). Ce processus est relativement lent et dépend des facteurs environnementaux et des microorganismes en présence.

Ces biofims sont omniprésents dans de nombreux domaines; dans lesquels ils présentent des risques sanitaires et causent des dommages relativement importants.

En santé humaine par exemple, les biofilms sont responsables d'infections de plus en plus difficiles à juguler : sur toute la sphère ORL (conduit auditif, muqueuse nasale, conjonctive de l'oeil ...), sur les dents (apparition de tartre, de caries, ...), sur les bronches, les poumons (chez les patients atteints de mucoviscidose ...), au niveau du tractus urogénital (...). Ils sont en outre à l'origine de la plupart des pathologies nosocomiales (plus de 10 000 décès par ans) en se formant au niveau de cathéters, ou d'implants (valves cardiaques, hanches artificielles, sondes urinaires,...) (J.W. Costerton, P. Stewart et E.P. Greenberg, Bacterial Biofilms : « A common cause of persistent infections ». Science, vol. 284, pep1318-1322).

Les biofilms concernent également l'industrie agro-alimentaire pour leur implication dans les cas d'intoxications alimentaires (formation lors de ruptures de la chaîne du froid, développement sur les outils de tranchage, de broyage, sur les surfaces de travail). Ils sont également présents dans les tours de réfrigération, responsables d'infections par les Legionelles.

Le développement et le comportement de ces biofilms restent cependant mal connus du fait de leur complexité à être étudiée, bien que de nombreuses méthodes d'étude du développement des biofilms sont mises en oeuvre.

Parmi les méthodes mises en oeuvre pour étudier le comportement de telles communautés microbiennes, on connaît celle décrite dans la demande de brevet internationale WO2005/090944. La méthode décrite dans cette demande de brevet repose sur la modélisation du développement des biofilms dans un milieu non homogène, trouble et opaque correspondant au milieu de culture dans lequel les microorganismes se développent pour former de tels biofilms. Cette modélisation est établie sur la mesure de la viscosité du milieu de culture. La notion de viscosité ne décrit que partiellement l'effet du biofilm. En effet, un biofilm est constitué :
- d'une part, par une quantité d'exopolysaccharide (EPS), ou autre substance visqueuse, sécrété par les microorganismes,
- et d'autre part, par un réseau, un maillage de fibres et de corps cellulaires. En effet, les microorganismes utilisent des appendices cellulaires du microorganisme (flagelles, pilis,....) pour adhérer sur les surfaces.

La mesure correspond plus spécifiquement à une mesure de viscosité et une mesure de résistance à la traction sur les appendices cellulaires. En effet, la mesure utilise des particules (ou billes) magnétisables, magnétiques, ou chargées électriquement (pouvant être magnétisées ou chargées électriquement sous l'effet d'un champ magnétique, électro-magnétique, ou électrique), ou recouverte d'au moins une couche magnétique ou magnétisable. Par la suite, dans le présent texte, on emploiera le terme " magnétique " en référence indifféremment à l'expression " chargée électriquement " ou aux termes " magnétique " ou " magnétisable " ou à l'expression " recouverte d'au moins une couche magnétique ou magnétisable ". Ces particules présentes sur la surface où va se développer le biofilm, vont être piégées par la substance visqueuse sécrétée par les microorganismes et par les appendices cellulaires utilisés par les microorganismes. Les particules sont immobilisées par ces deux facteurs, dans des proportions/rapports variables en fonction des microorganismes étudiés.

Ainsi, la méthode décrite dans la demande de brevet susmentionnée consiste à :
a) immerger au moins une particule magnétique dans un milieu de culture. Le milieu de culture est de préférence disposé dans un ou plusieurs puits d'une microplaque.
b) soumettre le milieu de culture à un champ magnétique, électrique, ou électromagnétique, de façon à mettre ladite particule en mouvement.
c) détecter le degré de liberté de mouvement de ladite particule dans ladite culture.

Le degré de mobilité des particules est réduit ou nul si la viscosité augmente suite à la production d'EPS par les microorganismes, ou si les microorganismes développent des appendices cellulaires (flagelles, pilis,....) pour adhérer sur la surface, piégeant les particules en même temps.

Cette dernière étape c) est réalisée de préférence par mesure optique. Il s'agit d'éclairer le fond des puits de la microplaque au moyen d'une source lumineuse de sorte à éclairer la ou les particule(s) magnétique(s), et ainsi à déterminer le mouvement de la ou des particule(s) dans le milieu de culture, par comparaison d'images. Cette comparaison s'effectue d'une part avant et après effet d'un champ magnétique, électrique, ou électromagnétique, et d'autre part à des intervalles de temps donnés (temps de développement microorganismes formant ou non un biofilm).

Les dispositifs de détection optique utilisés sont des dispositifs conventionnels de prise de vue d'image (scanner, appareil photo, caméra, ...). Les vues correspondent au fond de puits, observé par transparence par-dessous. Le chemin optique classique traverse successivement les éléments suivants :
- fond externe du puits,
- matériaux constituant le fond du puits (transparent : plastique, verre...),
- fond intérieur du puits,
- milieu de culture,
- ménisque milieu de culture,
- air au-dessus du milieu de culture.

Quel que soit le système de prise de vue, l'expérimentateur se trouve confronté à un problème majeur lié à la formation de reflets entre le fond du puits et la surface liquide du milieu de culture du fait de l'existence d'un ménisque du milieu de culture. L'image obtenue par le dispositif de détection optique est alors particulièrement complexe, voire parfois impossible à exploiter.

Outre la difficulté d'analyse de l'image liée au ménisque formé à la surface du milieu de culture, l'expérimentateur se trouve également confronté, lorsqu'il souhaite relever simultanément les images correspondant à chacun des puits de la microplaque, à un problème lié au parallaxe entre l'image des puits disposés au centre de la microplaque, et l'image des puits disposés au bord de la microplaque. En effet, si au centre, le fond du puits est aisément distinguable, il n'en est pas de même pour le fond du puits formant la périphérie de la microplaque. L'image obtenue est en effet altérée par l'image de la paroi du puits (face inférieure et face supérieure), et le décalage entre le fond du puits, la surface du ménisque formé par le liquide contenu dans le puits, et l'ouverture supérieure du puits. Ces dioptres air-plastique (ou verre ou tout autre matériau transparent), plastique (ou verre ou tout autre matériau transparent), milieu de culture, milieu de culture-air, créent une image composite d'autant plus complexe que la parallaxe est importante, source de reflets notamment sur la paroi de puits.

L'invention vise notamment à résoudre les difficultés rencontrées par l'expérimentateur lors de l'analyse des images obtenues du fond des puits en proposant un procédé de relevés d'images du fond des puits, et un équipement adapté, permettant de supprimer l'effet du ménisque, ou tout du moins d'en limiter la portée.

À cet effet, et selon un premier aspect, l'invention concerne un procédé de relevés d'images du fond d'une plaque pourvue d'au moins un puits contenant un milieu de culture de microorganismes, caractérisé en ce qu'il comprend les étapes consistant à :
i) former une couche opacifiante recouvrant la surface du milieu de culture de sorte à former un fond de lecture,
ii) réaliser des prises d'images du fond du puits au moyen d'un dispositif optique de prise de vue à des intervalles de temps définis.

Par le terme " fond ", doit s'entendre dans le présent texte la paroi inférieure du puits, la face de la paroi concernée étant indifféremment la face intérieure ou la face extérieure. Les particules observées sont du coté de la face supérieure (au fond du puits).

Dans le cadre de l'étude du développement d'un biofilm dans le milieu de culture, l'étape de prises d'images comprend une première prise d'image du fond du puits correspondant à une image de référence, puis au moins une deuxième prise d'images après l'application d'un champ magnétique, électrique ou électromagnétique sur le milieu de culture ledit milieu contenant au moins une particule magnétique, magnétisable, chargée électriquement, ou recouverte d'au moins une couche magnétique ou magnétisable.

De préférence, la couche opacifiante est réalisée par dépôt dans le puits d'une composition non miscible avec le milieu de culture.

Ainsi, par le dépôt d'un produit opacifiant et non miscible avec le milieu de culture à la surface dudit milieu, les reflets entre le fond des puits et la surface du liquide sont éliminés, le relevé d'images en terme de netteté s'en trouvant amélioré.

Avantageusement, la couche opacifiante est réalisée par dépôt dans le puits d'une composition présentant une densité inférieure à celle du milieu de culture, de sorte à rester en surface du milieu de culture.

Par ailleurs, afin d'optimiser la netteté de l'image, la couche opacifiante sera réalisée de sorte à former, à la surface du milieu de culture, une couche homogène et uniformément répartie.

La couche opacifiante formée sera avantageusement opaque, non réfléchissante, afin de supprimer l'effet "miroir" du ménisque.

De même, afin d'éviter toute interaction physique et/ou chimique avec le milieu de culture, il sera avantageux que la composition constituant la couche opacifiante soit inerte. De même, par égard au manipulateur, il sera préféré d'employer une composition non toxique.

Avantageusement, la couche opacifiante comprend une huile translucide contenant un pigment miscible ou des microparticules hydrofuges.

Selon une configuration préférée de invention, le procédé comprendra en outre, en vue de l'analyse des images relevées, une étape préalable de repérage du fond du puits de sorte à extraire des images prises par le dispositif optiques les images de la paroi du puits, et ainsi analyser uniquement les images du fond du puits. Ce repérage préalable du fond du puits permet ainsi de s'extraire du problème lié au parallaxe entre l'image des puits au centre de la microplaque, et l'image des puits au bord de la microplaque.

On décrit dans la présente une plaque comprenant une pluralité de puits destinés à recevoir des échantillons d'un milieu de culture de microorganismes, dans laquelle le fond des puits comporte des moyens de repérage permettant de repérer la périphérie du fond dudit puits.

De préférence, les moyens de repérage consistent en une pluralité d'empreintes situées sur le fond des puits et disposées dans le prolongement des parois latérales des puits.

La formation de repères, et en particulier d'empreintes, en creux ou en relief, sur le fond du puits, et dans le prolongement des parois latérales du puits, permet un meilleur repérage du fond du puits, et ainsi de s'extraire de la difficulté liée au parallaxe entre l'image des puits au centre de la microplaque, et l'image des puits au bord de la microplaque.

Avantageusement, le fond des puits comprend trois empreintes réparties selon un angle de 120 degrés.

Afin de permettre les prises de vues par le fond, il est prévu que les puits soient pourvus d'un fond transparent.

D'autres objets et avantages de l'invention apparaîtront au cours de la description qui suit, faite en référence aux dessins annexés, dans lesquels :
- la figure 1 illustre des puits contenant un milieu de culture recouvert ou non d'une couche opacifiante, et de l'image correspondante obtenue du fond des puits ;
- la figure 2 est une vue du fond d'un puits selon une configuration particulière de l'invention.

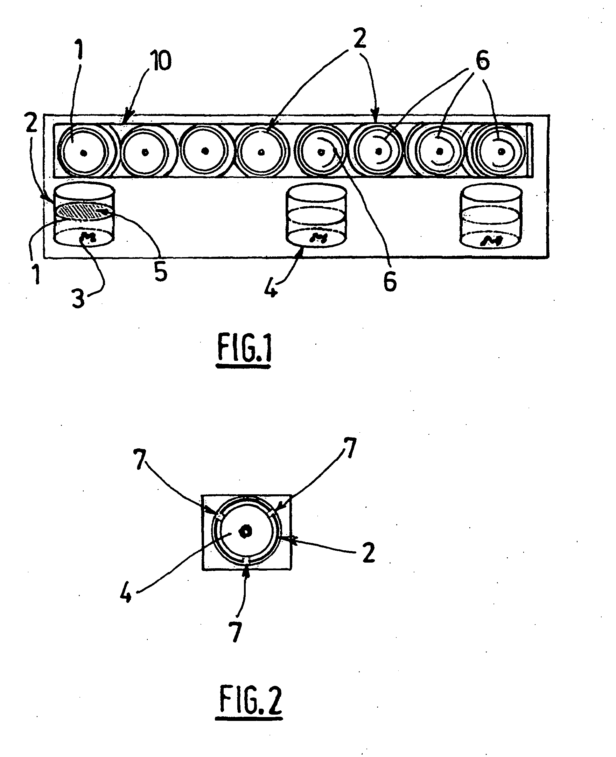

En relation avec les figures 1 et 2, il est décrit un équipement et un procédé permettant des relevés d'images d'un milieu de culture 1 et leur analyse en vue d'étudier le développement d'un biofilm dans ledit milieu 1.

Pour ce faire, l'équipement comprend une microplaque 10 comprenant des puits 2 dans lesquels est disposé du milieu de culture 1. Dans l'exemple illustré, la microplaque 10 comprend huit puits 2 alignés.

Des billes magnétiques 3 sont disposées au fond de chacun des puits 2. Ces billes magnétiques ont pour objet de permettre de mesurer la viscosité du milieu de culture en mesurant le degré de liberté des billes. En effet, suivant le degré de développement des microorganismes, le mouvement des billes magnétiques sous l'effet d'un champ magnétique, électrique, ou électromagnétique, sera plus ou moins entravé par le biofilm. Et à partir des mesures de la viscosité du milieu de culture à des intervalles de temps donnés, il pourra être procédé à la modélisation du développement et du comportement du biofilm présent dans le milieu de culture 1. Cette mesure est préférentiellement réalisée en comparant une prise de vue du fond du puits avant effet de champ magnétique, électrique ou électromagnétique, et une prise de vue après cet effet.

Afin d'autoriser la prise d'images par un dispositif optique, du type scanner, les puits 2 présentent avantageusement un fond 4 transparent.

Les biofilms se développant principalement dans un environnement aqueux, et adhérant aux surfaces submergées, en l'occurrence les billes magnétiques 3, le milieu de culture 1 choisi, dans l'exemple illustré, est l'eau.

Afin d'éliminer les reflets entre le fond des puits 2 et la surface du milieu de culture 1, perturbant le relevé et l'interprétation des images obtenues, une couche de fond de lecture 5 est disposée en surface dudit milieu de culture 1.

Cette couche de fond de lecture 5 est constituée d'une composition non miscible à l'eau, le milieu de culture 1 étant aqueux, et d'une densité inférieure à celle du milieu de culture 1. Ainsi, la couche de fond de lecture 5 reste i) parfaitement séparée du milieu de culture 1, et ii) au-dessus du ménisque, créant ainsi un fond d'image uni et opacifiant.

De préférence, on choisira une composition de couleur blanche, tel qu'un « pigment à l'huile » dilué dans l'huile, ou une composition dont la couleur contraste avec la couleur que l'on souhaite détecter dans les puits 2.

Par ailleurs, la composition choisie sera de préférence inerte chimiquement et physiquement vis-à-vis du milieu de culture 1, et non toxique afin d'en simplifier sa manipulation.

En outre, la composition déposée en surface du milieu de culture 1 devra de préférence constituer une couche opaque non réfléchissante, afin d'éliminer l'effet « miroir » du ménisque.

De préférence, mais non exclusivement, la composition comprend une huile translucide contenant un colorant miscible, sans coefficient de partage avec l'eau, ou bien une huile translucide mélangée par des microparticules hydrofuges, tel que du Téflon® micronisé.

Bien que de mise en oeuvre plus difficile, il pourra être également utilisé, pour constituer la couche opacifiante, des poudres hydrofuges ou des cires.

La couche de fond de lecture 5, ainsi constituée et formée à la surface du milieu de culture 1, permet d'éliminer les reflets formés par la surface du milieu de culture 1.

Après avoir procédé au dépôt de la composition dans le puits 2, et formé à la surface du milieu de culture 1 une couche opaque de préférence uniforme (sans surépaisseur) et recouvrant toute la surface dudit milieu 1, la microplaque 10 est positionnée au-dessus d'un scanner destiné à réaliser une première prise de vue du fond des puits 2 (image de référence), puis une pluralité de prises d'images, à des intervalles de temps donnés, après l'application d'un champ magnétique électrique ou électromagnétique pendant un temps donné (de l'ordre par exemple d'une minute) conformément au principe décrit dans la demande de brevet WO2005/090944. En comparant les prises de vues avec un logiciel d'analyse d'images, il est possible d'évaluer le degré d'immobilisation des particules. Si les particules sont mobiles (sans présence de microorganismes par exemple), elles forment un amas opaque au point de maximum d'intensité du champ magnétique (ou électrique ou électromagnétique). Si les particules sont immobilisées, on n'observe aucune différence entre les deux prises de vues. En fonction de l'évolution du développement du biofilm, on observe une évolution de la différence entre les deux prises de vues.

Sur la figure 1 est illustré les images du fond 4 des puits relevées par le scanner, les quatre puits 2 les plus à gauche contenant un milieu de culture 1 recouvert par une couche de fond de lecture 5, les quatre puits 2 les plus à droite contenant un milieu de culture 1 sans couche de fond de lecture.

L'image du fond 4 des quatre puits 2 les plus à droite montre clairement la présence du ménisque 6 rendant l'interprétation de l'image relevée difficile, alors qu'aucun ménisque n'apparaît lorsque les puits comportent une couche de lecture de fond 5 (cf. les quatre puits de gauche).

L'interprétation d'une image lorsque le puits ne comporte pas de couche de lecture de fond 5 est rendue d'autant plus difficile qu'au problème de la présence de ménisque s'ajoute le problème lié à l'effet du parallaxe, lequel est maximal au bord de la microplaque.

Aussi, et afin de palier ce dernier problème, il sera avantageux de prévoir des puits 2 dont le fond 4 présente, dans le prolongement des parois latérales, des empreintes 7 (cf. figure 2).

Selon une configuration préférée de l'invention, le fond 4 des puits 2 comprend trois empreintes écartées les unes des autres selon un angle de 120 degrés, de sorte à délimiter clairement le fond 4 des puits 2.

Avantageusement, les empreintes 7 consistent en des creux de forme pyramidale.

L'invention est décrite dans ce qui précède à titre d'exemple. Il est entendu que l'homme du métier est à même de réaliser différentes variantes de l'invention sans pour autant sortir de l'invention.

## Revendications

1. Procédé de relevés d'images du fond d'une plaque pourvue d'au moins un puits (2) contenant un milieu de culture (1) de microorganismes, **caractérisé en ce qu'**il comprend les étapes consistant à :
i) former une couche opacifiante (5) recouvrant la surface du milieu de culture (1) de sorte à former un fond de lecture,
ii) réaliser des prises d'images, à des intervalles de temps définis, du fond du puits (2) au moyen d'un dispositif optique de prise de vue.

2. Procédé de relevés d'images selon la revendication 1, **caractérisé en ce que** l'étape de prises d'images comprend une première prise d'image du fond du puits (2) correspondant à une image de référence, puis au moins une deuxième prise d'images après l'application d'un champ magnétique, électrique ou électromagnétique sur le milieu de culture (1), ledit milieu contenant au moins une particule magnétique, magnétisable, chargée électriquement, ou recouverte d'au moins une couche magnétique ou magnétisable.

3. Procédé de relevés d'images selon la revendication 1 ou la revendication 2, **caractérisé en ce que** la couche opacifiante (5) est réalisée par dépôt dans le puits (2) d'une composition non miscible avec le milieu de culture (1).

4. Procédé de relevés d'images selon l'une quelconque des revendications 1 à 3, **caractérisé en ce que** la couche opacifiante (5) est réalisée par dépôt dans le puits (2) d'une composition présentant une densité inférieure à celle du milieu de culture (1).

5. Procédé de relevés d'images selon l'une quelconque des revendications 1 à 4, **caractérisé en ce que** la couche opacifiante (5) est réalisée de sorte à former, à la surface du milieu de culture (1), une couche homogène et uniformément répartie.

6. Procédé de relevés d'images selon l'une quelconque des revendications 1 à 5, **caractérisé en ce que** la couche opacifiante (5) formée est une couche opaque, non réfléchissante.

7. Procédé de relevés d'images selon l'une quelconque des revendications 1 à 6, **caractérisé en ce que** la couche opacifiante (5) formée est inerte.

8. Procédé de relevés d'images selon l'une quelconque des revendications 1 à 7, **caractérisé en ce que** la couche opacifiante (5) comprend une huile minérale translucide contenant un pigment miscible.

9. Procédé de relevés d'images selon l'une quelconque des revendications 1 à 7, **caractérisé en ce que** la couche opacifiante (5) comprend une huile translucide contenant des microparticules hydrofuges.

10. Procédé de relevés d'images selon l'une quelconque des revendications précédentes, **caractérisé en ce que**, en vue de l'analyse des images relevées, on procède à un repérage du fond (4) du puits (2) de sorte que seules les images correspondant au fond (4) du puits (2) sont analysées.

## Claims

1. A method for reading images of a base of a plate provided with at least one well (2) containing a culture medium (1) of microorganisms **characterized in that** it consist of:
i) forming an opaquing layer (5) covering a surface of the culture medium (1) to form a reading base, and
ii) imaging, at specific time intervals, the base of the well (2) using an imaging optical device.

2. The method according to claim 1, **characterized in that** the imaging step comprises a first imaging of the base of the well (2) corresponding to a reference image, then at least one second imaging after application of a magnetic, electric or electromagnetic field on the culture medium (1), the medium containing at least one particle which is magnetic, magnetisable, electrically loaded or covered with at least one magnetic or magnetisable layer.

3. The method according to claim 1 or claim 2, **characterized in that** the opaquing layer (5) is made by deposition into the well (2) of a composition not miscible with the culture medium (1).

4. The method according to anyone of claims 1 to 3, **characterized in that** the opaquing layer (5) is made by deposition into the well (2) of a composition having a density lower than that of the culture medium (1).

5. The method according to anyone of claims 1 to 4, **characterized in that** the opaquing layer (5) is made to form at the surface of the culture medium (1), a homogeneous and uniformly distributed layer.

6. The method according to anyone of claims 1 to 5, **characterized in that** the opaquing layer (5) formed is a non-reflecting opaque layer.

7. The method according to anyone of claims 1 to 6, **characterized in that** the opaquing layer (5) formed is inert.

8. The method according to anyone of claims 1 to 7, **characterized in that** the opaquing layer (5) comprises a translucent mineral oil containing a miscible pigment.

9. The method according to anyone of claims 1 to 7, **characterized in that** the opaquing layer (5) comprises a translucent oil containing water-repellent microparticles.

10. The method according to anyone of preceding claims, **characterized in that**, to analyze the developed images, the base (4) of the well (2) is located so that only images corresponding to the base (4) of the well (2) are analyzed.

## Patentansprüche

1. Verfahren zum Lesen von Bildern des Grunds einer Platte, die mit mindestens einem das Kulturmedium (1) für Mikroorganismen enthaltenden Schacht (2) ausgestattet ist, **dadurch gekennzeichnet, dass** es aus folgenden Schritten besteht:
i) Bilden einer trübenden Schicht (5), welche die Oberfläche des Kulturmediums (1) zur Bildung eines Lesegrunds bedeckt und
ii) Durchführung von Aufnahmen des Schachtgrunds (2) in definierten Zeitabständen unter Verwendung einer optischen Aufnahmevorrichtung.

2. Verfahren zum Lesen von Bildern nach Anspruch 1, **dadurch gekennzeichnet, dass** der Aufnahmeschritt die Aufnahme eines ersten Bildes des Schachtgrunds (2) umfasst, das einem Referenzbild entspricht, worauf mindestens eine zweite Aufnahme folgt nach Anwendung eines magnetischen, elektrischen Feldes oder elektromagnetischen Feldes auf das Kulturmedium (1), wobei besagtes Medium mindestens ein magnetisches, magnetisierbares, elektrisch geladenes Teilchen enthält oder mit mindestens einer magnetischen oder magnetisierbaren Schicht bedeckt ist.

3. Verfahren zum Lesen von Bildern nach Anspruch 1 oder Anspruch 2, **dadurch gekennzeichnet, dass** die trübende Schicht (5) durch Ablage im Schacht (2) einer nicht mit dem Kulturmedium (1) mischbaren Zusammensetzung erhalten wird.

4. Verfahren zum Lesen von Bildern nach einem beliebigen der Ansprüche 1 bis 3, **dadurch gekennzeichnet, dass** die trübende Schicht (5) durch Ablage im Schacht (2) einer Zusammensetzung, die eine geringere Dichte als das Kulturmedium (1) aufweist, erhalten wird.

5. Verfahren zum Lesen von Bildern nach einem beliebigen der Ansprüche 1 bis 4, **dadurch gekennzeichnet, dass** die trübende Schicht (5) derart gebildet wird, dass an der Oberfläche des Kulturmediums (1) eine homogene und gleichmäßig verteilte Schicht gebildet wird.

6. Verfahren zum Lesen von Bildern nach einem beliebigen der Ansprüche 1 bis 5, **dadurch gekennzeichnet, dass** die gebildete trübende Schicht (5) eine undurchsichtige, nicht reflektierende Schicht ist.

7. Verfahren zum Lesen von Bildern nach einem beliebigen der Ansprüche 1 bis 6, **dadurch gekennzeichnet, dass** die gebildete trübende Schicht (5) inert ist.

8. Verfahren zum Lesen von Bildern nach einem beliebigen der Ansprüche 1 bis 7, **dadurch gekennzeichnet, dass** die trübende Schicht (5) ein lichtdurchlässiges Mineralöl enthält, das ein mischbares Pigment enthält.

9. Verfahren zum Lesen von Bildern nach einem beliebigen der Ansprüche 1 bis 7, **dadurch gekennzeichnet, dass** die trübende Schicht (5) ein lichtdurchlässiges Öl enthält, das wasserabweisende Mikropartikel enthält.

10. Verfahren zum Lesen von Bildern nach einem beliebigen der vorausgehenden Ansprüche, **dadurch gekennzeichnet dass** zur Analyse der entwickelten Bilder der Grund (4) des Schachts (2) derart eingerichtet ist, dass nur Bilder analysiert werden, die dem Grund (4) des Schachts (2) entsprechen.
